# EUROPEAN PATENT APPLICATION

(11) **EP 4 015 621 A1**
(43) Date of publication of application: **22.06.2022**
(21) Application number: 20215146.0
(22) Date of filing: 17.12.2020
(51) Int. Cl.: C12M 3/00, C12M 1/12, C12M 1/00, C12M 1/26, C12M 1/24, C12M 3/04

(54) **DEVICE FOR SEEDING CELLS**

(71) Applicant: abc biopply ag, 4500 Solothurn (CH)
(72) Inventor: FAISST, Arne, 4500 Solothurn (CH); LEU, Marco, 4500 Solothurn (CH); THOMSEN, Andreas, 4500 Solothurn (CH)
(74) Representative: Frei Patent Attorneys

(57) **Abstract**

A device for seeding cells comprises a container with a wall, a bottom and a lid. The wall extends between the bottom and the lid. The container can be equipped to be loaded with cells, in particular with cells form a cell suspension. The container comprises a rotation axis. The device is further equipped to rotate the container around the rotation axis, by means for rotating the container about said rotation axis.

The container comprises a structured surface. The structured surface can be arranged at the inner surface of the container. The structured surface comprises structures. These structures are equipped to receive the cells. The rotation exerts a (g-)force in direction of the structured surface, this means that the g-force acts perpendicular to the structured surface. The exerted force in the direction of the structured surface resembles a g-force required for sedimentation of the cells into the structures.

## Description

The invention relates to the field of cell biology and three-dimensional (3D) aggregates of cells. It relates to a device for seeding cells, a serial reactor, a cartridge, the use of the device for generating, maintaining and/or harvesting of 3D cell aggregates and/or for the production of conditioned media, a method to generate 3D cell aggregates and a method for producing conditioned medium produced by 3D cell aggregates as described in the preamble of the independent claims.

First approaches to grow mammalian cells under laboratory conditions date back more than one century, as described in CARREL A. CULTIVATION OF ADULT TISSUES AND ORGANS OUTSIDE OF THE BODY. JAMA 1910;55 (16):1379. Due to development of refined methods, culture media and supplements, cell cultures have since become indispensable models for both healthy and diseased tissues and cells. The traditional type of culture vessel comprises at least one planar surface, to which cells adhere and grow as monolayer or 2-dimensional culture. However, as such culture conditions are highly artificial and lack several properties of the original tissue, it is now generally accepted that 3-dimensional cell cultures represent a more physiological model. Therefore, experiments performed with 3D cultures are more likely to yield results that reflect the situation in vivo, as described in Mittler F, Obeïd P, Rulina AV, et al. High-Content Monitoring of Drug Effects in a 3D Spheroid Model. Frontiers in oncology 2017;7:293.

Several methods to generate 3-dimensional cell cultures have been introduced in the past 50 years, as described in Sutherland RM, McCredie JA, Inch WR. Growth of multicell spheroids in tissue culture as a model of nodular carcinomas. Journal of the National Cancer Institute 1971;46 (1):113-20, Tomasi RF-X, Sart S, Champetier T, et al. Individual Control and Quantification of 3D Spheroids in a High-Density Microfluidic Droplet Array. Cell reports 2020;31 (8):107670 or Sutherland RM, Sordat B, Bamat J, et al. Oxygenation and differentiation in multicellular spheroids of human colon carcinoma. Cancer research 1986;46 (10):5320-9. Nevertheless, the broad majority of drug screening experiments is still performed using 2D (monolayer) cultures.

The background for this dilemma is multifaceted and mainly due to practical and/or technical obstacles. As an example, a commercial market survey as described in Ryan S-L, Baird A-M, Vaz G, et al. Drug Discovery Approaches Utilizing Three-Dimensional Cell Culture. Assay and drug development technologies 2016;14 (1):19-28 states
"...found that there was no complete system, no one relatively simple method that could transform single cells into robust 3D structures, which could be easily manipulated, treated, imaged, analysed, and up scaled.

The survey highlighted issues such as (i) poor reproducibility, (ii) labor intensiveness, (iii) overcomplicated systems, (iv) inability to upscale, (v) difficulty in recovering cultures, (vi) incompatibility with automated systems, (vii) lack of flexibility, (viii) poor imaging quality, and (ix) long-term stability problems".

Recently, a combined approach has been established and evaluated, using two steps:
- Multicellular aggregates (micro tissues) are generated in micro structured hydrogel chips, as described in Thomsen AR, Aldrian C, Bronsert P, et al. A deep conical agarose micro-well array for adhesion independent three-dimensional cell culture and dynamic volume measurement. Lab on a chip 2017;18 (1):179-89.
- Micro tissues are harvested from hydrogel chips and sorted into standard multi well plates.
This procedure yielded a high level of homogeneity among individual micro wells and among plates, as described in Marco P. Leu et al. Easy mass production of homogenous and uniform 3D spheroids for high-throughput screening applications. (available at: https://biopply.com/assets/References/MPLeu-et-al-V17-20200703.pdf - accessed October 14, 2020).

The basic principle is related to a modular, scaffold-free 3D cell culture platform using conical hydrogel micro wells, as described in WO 2014/07243. Products based on these conical hydrogel micro wells have been shown to be a versatile and efficient tool for cancer cell investigations in vitro, as described in Voglstaetter M, Thomsen AR, Nouvel J, et al. Tspan8 is expressed in breast cancer and regulates E-cadherin/catenin signalling and metastasis accompanied by increased circulating extracellular vesicles. The Journal of pathology 2019;248 (4):421-37, Oria VO, Bronsert P, Thomsen AR, et al. Proteome Profiling of Primary Pancreatic Ductal Adenocarcinomas Undergoing Additive Chemoradiation Link ALDH1A1 to Early Local Recurrence and Chemoradiation Resistance. Translational oncology 2018;11 (6):1307-22 and Mohamed AA, Thomsen A, Folio M, et al. FAK inhibition radiosensitizes pancreatic ductal adenocarcinoma cells in vitro. Strahlentherapie und Onkologie Organ der Deutschen Rontgengesellschaft ... [et al] 2020. The generation of extracellular vesicles (EVs) from multicellular aggregates was significantly more efficient than in monolayer cultures, as described in Rocha S, Carvalho J, Oliveira P, et al. 3D Cellular Architecture Affects MicroRNA and Protein Cargo of Extracellular Vesicles. Advanced science (Weinheim, Baden-Wurttemberg, Germany) 2019;6 (4):1800948.

The sedimentation of cells is known e.g. from US 2010/184222 and cell cultivation under rotation is known e.g. from WO9728252 or US 5,858,783, were a micro gravity environment is created. The reduction of the acting gravity in a micro gravity environment can prevent the attachment of the cells to the wall of a reactor.

It is therefore an object of the invention to provide an improved device for seeding cells, a serial reactor, a cartridge, the use of the device for generating, maintaining and/or harvesting of 3D cell aggregates and/or for the production of conditioned media, a method to generate 3D cell aggregates and a method for producing conditioned medium produced by 3D cell aggregates which enable a large-scale harvesting and/or generation of 3D cell aggregates.
These objects are achieved by a device and a method according to the independent claims.

The device for seeding cells comprises a container with a wall, a bottom and a lid. The wall can be regarded as a mantle structure and can be designed as an outer shell. The wall can be designed as a cylindrical tube. The wall extends between the bottom and the lid. The container circumscribes a cavity confined by the wall, the bottom and the lid. The container can be equipped to be loaded with cells, in particular with cells forming a cell suspension.
The cell suspension comprises cells suspended in culture medium. In other words: The cell suspension is a solution with suspended cells in culture medium. The culture medium can be any kind of physiological medium. The cell suspension can be a homogenous suspension. The cell suspension can comprise the cells which are to be aggregated to 3D cell aggregates. These 3D cell aggregates can also be called spheroids and/or organoids. The 3D cell aggregates can be 3D structures of cells.

The container comprises a rotation axis. The rotation axis can be aligned in a way that it passes more specifically extents through the bottom and the lid. The rotation axis can be oriented horizontally or vertically with respect to the direction of gravity. In some embodiments the device is equipped to arbitrary position the rotation axis with respect to the direction of gravity.

The device is further equipped to rotate the container around the rotation axis, by means for rotating the container about said rotation axis. In particular the device can be equipped to controllably rotate the container.

The rotation of the device can be adjusted to the requirements of the cells and the 3D cell aggregates which might be grown from the seeded cells. The corresponding adjustment might be regulated by controlling the rotation of the container.

The container comprises a structured surface. The structured surface can be arranged at the inner surface of the container. The structured surface comprises structures. These structures are equipped to receive the cells.

In an embodiment of the invention the structured surface can be arranged at the wall of the container, in particular at the inner surface of the wall of the cavity. In another embodiment the structured surface can be arranged at the bottom and/or the lid with the rotation axis essentially running parallel to the bottom and/or the lid.

The structured surface is equipped to receive cells. The cells can move into the structures of the structured surface. The cells are received in the structured surface and can be collected and/or picked up by the structures. The cells can be accommodated in the structures of the structured surface. In other words, the structured surface provides a housing for the cells. The cells are mainly not repelled form the wall of the wall, as taught e.g. in WO 97/28252.

The structured surface can be arranged essentially parallel to the rotation axis. The rotation of the container around the rotation axis exerts a (g-)force in direction of the structured surface, this means that the g-force acts perpendicular to the structured surface. The exerted force in the direction of the structured surface resembles a g-force required for sedimentation of the cells into the structures. The g-force can be a standard gravitational force. Accordingly, a rotation around the rotation axis can allow for a sedimentation like receiving of the cells in the structured surface. In other words: The rotation around the rotation axis can allow for the sedimentation of the cells in the structured surface under gravitation-like conditions. The sedimentation by gravity corresponds to a compacting of the cells through centrifugation.

In some embodiments the structured surface is not arranged parallel to the rotation axis. In these embodiments the structured surface can be arranged in an angle with respect to the rotation axis, as e.g. in a conical container. This enables for a gradual distribution of the cell along the rotation axis. A gradient of the cell distribution depends on the angle between the structured surface and the rotation axis. The gradient in the cell distribution can lead to a distribution of the size of 3D cell aggregates.

Before and/or during seeding the rotational speed can be varied. After seeding the rotational speed can be adjusted to exert a force of 1 g onto the cells in the structures of the structured surface to allow for standard gravitational conditions for the cells to grow. Accordingly, a variation of the g-forces might be advantageous while seeding the cells in the structures of the structured surface. For maintaining/culturing of 3D cell aggregates standard g-forces can be exerted onto the cells.

In an embodiment the centrifugal g-forces exerted onto the structured surface is essentially perpendicular to the structures of the structured surface. This might allow for an efficient sedimentation/compacting of the cells into the structures through centrifugation.

The cells seeded in the device might be biological cells but might also be inorganic cells, in particular the cells might particles comprising organic and/or inoganic matter. The cell is a basic structural and functional unit. The cells might be regarded as particles and/or particle like. The cells might be cells to be grown in a culture medium. The cells might be able to generate cell aggregates in particular 3D cell aggregates, meaning 3D aggregates of cells. The cells can be eukaryotic cells, in particular eukaryotic cells of animal and human origin, bacteria, fungi and/or a mixture thereof. The cells can be a mixture of different types biological cell and/or non-living particles. Non-living particles or cells might be ECM-particles (extracellular matrix particle), which can enhance the aggregation of cells.

The cells can be selected from the group of biological and functional units of multicellular organisms, primary cells, that were recently removed from the healthy or diseased organism, cells that have been subjected to manipulations and selecting steps after original isolation, e.g. expansion of cell number in vitro or in vivo, selection of subpopulations with special properties, treatment with compounds or growth factors, exposure to physical effects, labelling of cells or parts thereof, or application of genetic modifications. The skilled person familiar with cell biology and cell culture methods will gather from these examples that cells may have been subjected to a broad spectrum of treatments. The cells might be kept in a suspension while loading. The cells might be in a single cell suspension.

The device for seeding cells can be a reactor, in particular a bioreactor, meaning a device for cultivating cells at optimal, controlled conditions. The reactor in particular the bioreactor can support an active environment. The reactor in particular the bioreactor can be designed to grow cells.
Cell aggregates, in particular 3D cell aggregates, can be an accumulation of two or more cells. The accumulation can include both cell-to-cell adhesion, as it takes place in spheroid formation, and adhesion- independent accumulation of cells. The term "3D cell aggregates" is directed to cells present in a three-dimensional shape and not in the form of a mono-layer (i.e. a two-dimensional shape).
The device for seeding, might also be called seeding device, can be used to generate uniform or rather homogenous cell aggregates. The term "uniform" refers to substantially identical (particularly with respect to the size) cell aggregates. The uniformity of the 3D cell aggregates can be related to the cross-section area, circumference, roughness and/or roundness of the 3D cell aggregates.

The size of the container can largely vary from small cylinders (e.g. in the size of a 50 mL standard 'bluecap' centrifuge tube) up to large bioreactors with a volume of several hundreds of litres and an inner surface of a few m².

The device can be used as a closed system, which eliminates the risk of microbial contamination, and which means that hygienic requirements to the laboratory facilities are less critical.

The structured surface can comprise a hydrogel and can be called structured hydrogel. The structured hydrogel can be arranged at an inner surface of device or rather the container. In an embodiment of the invention a hydrogel is used in the structured surface.
The hydrogel can be cell repellent. This can enable the formation of uniform 3D cell aggregates because essentially all cells reach the structures of the structured surface. Single cells do not attach to the sidewalls or the bottom of structures.
The hydrogel is permeable to metabolites, nutrients, oxygen and medium, this enables a continuous supply of all components to the cells. Furthermore, receiving of the cells in a structured hydrogel enables to reduce a wall effect, were the cells might not be sufficiently supplied with nutrient and other compounds of the medium, as the wall comprising the structured surface comprises the hydrogel. In other words: Receiving the cells in a structured, permeable hydrogel prevents withholding of metabolites, nutrients, oxygen and medium from the cells as it may occurs in structured surfaces made of none permeable material. Accordingly, the hydrogel at the wall of the containment structure enables for an essentially continuous supply of nutrients. Furthermore, due to the application of g-forces in the direction of the structured hydrogel a dynamic pressure might be exerted onto cells received in the structures of the structured surface. The usage of hydrogel in the structured surface can substantially reduce the dynamic pressure, as it is permeable to the medium. Furthermore, the structured hydrogel might enable for a full or rather complete supply of the cells with the medium. The structured hydrogel can be perfused by the medium.

The structured surface can be made of various material. The material of the structured surface can be open for diffusion to ensure a (steady) supply of the cells seeded in/on the structured surface. Additional to or instead of hydrogel also a micro- and/or nanoporous polymer with interconnecting pores, as in particular very fine fleece, such as in particular porous Teflon membrane, can be used. Alternatively, a fleece made of polyethylene fibres, which might be not cell-adhesive, in particular material like Tyvek^{®} can be used as material of the structured surface.
The material of the structured surface can enhance mechanical stability and storability of the structured surface, wherein drying out of the material might not be an issue.

The structured surface can comprise micro wells for receiving cells. The microwells can be called structures of the structured surface. This enables for safely accommodating the cells on the structured surface.

The structured surface can cover essentially the whole wall of the container, in particular the inner wall meaning the inner surface can essentially be completely covered by the structured surface. The inner wall might be directed such that it faces towards the rotation axis.
Due to the complete coverage of the wall of the container homogenous seeding and/or growing conditions for the cells and/or 3D cell aggregates can be provided. A reduction of the coverage of the wall can lead to a generation of 2D cell cultures. This might influence the harvesting of the 3D cell aggregates and/or a production of conditioned media. An unregulated formation of 2D cell layers, as alien/foreign aggregates, in the container can lead to a contamination of the harvested cell aggregates and/or conditioned medium.
In an embodiment at least a portion of the inner wall of the container not covered by the structured surface can be coated with a cell repellent to avoid growth off two-dimensional cell aggregates in the container. In other words: such that the portion of the wall not covered by the structured surface is coated with a cell repellent. This enables for a harvesting of 3D cell aggregates and/or a production of conditioned media being undisturbed from 2D cell aggregates. In other words: In an embodiment the wall of the container is at least partially equipped to repel cells. Accordingly, cells not received by the structured surface do not (or negligibly) grow in the container, and/or cells are inhibited to form 2D cell cultures. In other embodiments the whole inner wall of the container can be coated with a cell repellent to avoid growth off two-dimensional cell aggregates in the whole container, even below the structured surface.

In another embodiment 2D cell layer formation is promoted on the inner wall of the container and the structured surface is arranged on top of the 2D cell layer, e.g. in form of a cartridge or the like. This enables for distance co-cultures.

The device can be equipped to distribute, in particular homogenously distribute, the cells in the structures of the structured surface. The structures might be designed as at least one of grooves, notches, holes, cavities, gaps, perforations, punctations, indentations, depressions, and recessions.
The distribution or rather homogenous and/or uniform distribution of the cells in/on the structured surface enables for essentially uniform 3D cell aggregates. The 3D cell aggregates can be essentially uniform in terms of size and/or homogeneous in terms of composition.

The container can comprise an inner cavity, also called tubing, wherein cells are homogenously emitted from inner cavity towards the structured surface. The inner cavity can be designed as an inner tubing.

The inner cavity/tubing is different from the above described cavity confined by the wall, the bottom and the lid. The inner cavity/tubing is positioned inside the container. The inner cavity/tubing is surrounded by the wall, the bottom and the lid. In other wards: The inner tubing is an additional cavity inside the container.
The inner tubing can essentially extend from the bottom to the lid. The inner tubing can be punctured and/or perforated. The cells can be injected inside the container via the inner tubing.

The inner tubing can rotate together with and/or in a different speed and/or in a different direction than the container. In another embodiment of the invention the inner tubing is stationary and does not rotate.

The device can be equipped to generate 4-6400, in particular 4-400, in particular 4-2000, in particular 4-2500, aggregates/cm² of the structured surface. The aggregates can be 3D cell aggregates. Accordingly, the device can be equipped to generate 4-6400, in particular 4-400, in particular 4-2000, in particular 4-2500, aggregates of cells per square centimetre of the structured surface.

The device can be equipped to generate at least 10'000 3D cell aggregates per batch and/or device. The device can generate 10'000 - 1'000'000 or more aggregates of cells per batch and/or device.

The number of generated/formed 3D cell aggregates per cm² can depend on the spacing of the structured surface. The numbers of structures can range from 4 structures/cm² up to 2,500 structures/cm². For generation of large or very small micro tissues, structure spacing may also be chosen above or below this range.

The container can be symmetrical with respect to the rotation axis. The container can be rotationally symmetric, a cylindrical container and/or a conical container. In the cylindrical container the structured surface or the inner surface of the container (on which the structured surface can be arranged) can be arranged essentially parallel to the rotation axis. In the conical container the structured surface or the inner surface of the container (on which the structured surface can be arranged) not arranged parallel to the rotation axis.
The conical container can comprise a central rotation axis. In the conical container a gradient in the distribution of the cells in the structured surface can be obtained. This can enable to provide a distribution of size of the 3D cell aggregates. Meaning, that smaller and larger 3D cell aggregates can be obtained in the conical container, depending on the slant/slope of the cone, as described above.

The device can comprise at least one of the following features:
- the wall of the container is curved and/or bent;
- the structured surface is curved;
- the container, in particular the wall/inner wall of the container, is cylindrical, conical and/or can have another regular or irregular shape;
- the device comprises a closing mechanism to removable connect the bottom and/or the lid to the wall.

The curvature of the container wall and/or the structured surface enables for an alignment g-forces essentially perpendicular to the structured surface. In particular the exerted g-force is aligned essentially perpendicular to essentially each position of the structured surface. In other words: the device can be equipped to align the exerted g-forces essentially perpendicular to the structured surface. This can enable for a rather uniform distribution of the cells in the structures of the structured surface and therefore can promote a uniform/homogenous manufacturing of 3D cell aggregates.

Due to the curvature of the container and the structured surface relative to the rotation axis essentially all structures, also called microwells, are essentially oriented perpendicular to the rotation axis. This means that the essentially perpendicular to the structured surface acting g-force acts uniformly on the structured surface and the cells loaded in the container. This allows for an essentially uniform sedimentation/compacting vector angle which is essentially parallel to the g-force. The uniform compacting angle/vector is perpendicular to the rotation axis and in direction of the acting g-force. Furthermore, the fluid column "above" each structure of the structured surface is identical for each structure. (The term "above" in this sense meaning, the fluid column perpendicular to the structured surface.) This allows for a uniform distribution of the cells in the structures of the structured surface. In other words: After seeding in cells are uniformly distributed in the structures of the structured surface due to the curvature of the structured surface and its orientation relative to the rotation axis and the acting g-force.

The closing mechanism might be designed as a reversible closing mechanism e.g. as thread, a bayonet lock, a flange, snap lock. Instead of a reversible closing mechanism, the lid might also be fixed in a permanent way with a permanent closing mechanism, e.g. by using glue, a welding procedure, a snap lock or a press-fit. Any other closing mechanism enabling the closure of the container might be used.

The device may further comprise at least one port to load the container with medium and/or cell suspension. This enables an easy access to the culture in the container, allowing the growth medium to be varied for optimum performance.
The port can be connected to at least on of
- a unidirectional valve to avoid contamination and/or to control fluid flow;
- a filter that retains unwanted cells, microbes and/or particles; and
- a silicone rubber membrane, which is open to diffusion of oxygen, so that cells are supplied properly.
In addition, the port can be closed with a cap that includes a silicone rubber septum. The septum may be punctured with an appropriate canula for injection and/or removal of medium.
The device can comprise at least one pump to feed medium and/or cell suspension into the container. The device can be thermally isolated to keep the temperature of the medium in the container constant. The device can also comprise a sensor, control and/or monitoring system to monitor, adjust and/or control the parameter in the container.

The dimension of the container can be such that the diameter of the container is larger that the height of the container. In particular the diameter can be at least three times larger than the height of the container. The height of the container can run essentially along the rotation axis, and the diameter of the container can be essentially perpendicular to the height of the container.
The height of the container extends from the bottom to the lid, in particular parallel to the rotation axis.

The device can be equipped for loading the container with cells while the container is rotating. This enables a homogenous distribution of cells in the cell suspension even during loading.
The orientation of rotation axis of the container during loading might be horizontally, vertically or arbitrary with respect to the earth gravity field. The orientation of the rotation axis can be adapted to the requirements of the loading, the cell suspension, the whole system, etc. A vertical rotation of the container around the rotation axis might be preferred while loading the cells in the container. In the vertical orientation of the rotation axis the container is arranged essentially vertically with respect to the earth gravitational force. In the vertical oriented container essentially no seeding of the cells occurs due to the earth gravitational force.
It is possible that the container and/or the rotation axis can be (arbitrary) moved during loading of the container. It can enable to inhibit a seeding of the cells while loading. The orientation of the rotation axis might be adjusted after loading for seeding the cells in the structured surface.

The device can be equipped to exchange culture medium in the container. This allows for a proper nutrient supply of the cells in the container. The device can comprise at least one of the following features:
- the device is equipped for exchanging the culture medium in an automated way;
- the device is equipped for exchanging the culture medium in a continuous or intermitted manner;
- the device is equipped for exchanging the culture medium while the container is rotating.

The resulting improved nutrient supply of the cells can lead to effective growth of the cells and the generation of 3D cell aggregates.

The container might be rotated while the culture medium is exchanged. A tubing might be used for injecting the medium into the container. As the tubing might not rotate while exchanging the medium for practical reasons a junction might be provided between the container and the tubing. The junction can be resistant to rotation and can be air- and/or waterproof.

The device can be equipped to exert g-forces of at least 0.1 g in direction of the structured surface. In particular the g-force exerted in direction of the structured surface can be at least one of at least 0.5, at least 1 g, at least 5 g, at least 10 g, and at least 300 g, at least 5000 g. The exerted g-force depends on the rotation speed of the container. The biologically relevant range of g-forces exerted to the cells is 0.1 g - 300 g. 1 g equals to the gravity of earth and is defined as 9.81 m/s² = 4 π² r3 n², with r being the radius of the container and n being the rotational speed - number of rotations of the container per second.
The exertion of the g-force to cells loaded into the container causes a "sedimentation" of the cells in the structures of the structured surface. A higher g-force causes faster sedimentation/compacting of the cells as compared to lower g-forces, where sedimentation/compacting is reduced. Accordingly, the speed of the "sedimentation" of the cells towards the structured surface can be regulated by the rotational speed of the container. After the sedimentation/compacting and therefore seeding of the cells is completed, the force exerted to the cells is preferably adjusted to 1 g, in order to provide standard growth conditions for the 3D cell aggregates. Nevertheless, it might be possible to exert a force different from 1 g to the seeded cells, depending on the requirements of the system.

A serial reactor comprises a first device as described above, a second device as described above and an adaptor. The adaptor is equipped to connect a first device to a second device. The serial reactor can also be called serial 3D reactors or communicator. Due to the connection of the first and second device a communication between the two devices a communication between the at least two devices is possible. This enables culture medium to flow from first device to the second device. This makes it possible to provide conditioned medium from the first device for the cell growth in the second device. The serial reactor can also enable the production of large quantities of conditioned medium.

In further embodiments a series of devices as described above can be connected to each other in a serial, communicating manner, where culture medium is exchanged between the several containers of the individual devices. In other words: More than two devices as described above can be connected to each other. The series of devices can be a serial reactor.
In the serial reactor the separate devices and/or container can be identical. The devices can be loaded with the same type of cells, e.g. for the large-scale production of conditioned medium. In another embodiment, the devices can be loaded with different types of cells, e.g. for adding further communication effects. In other words: The serial reactor can be loaded with/generate the same type and size of cell aggregates, but may also comprise different types of cells, so that the system represents a 'distant coculture'. Such a setup may be advantageous to produce conditioned cell culture media with a high concentration of factors secreted by the same and/or divers aggregates.

The devices or rather the individual container can be loaded and/or connected individually. This can allow for a continuous operation of the serial reactor, if needed.

The structured surface can comprise a surface, at least a first recession and a second recession for receiving cells for generating and cultivating cell aggregates. The first and the second recessions are spatially separated, and the surface between the first and the second recession is a sloped surface. The first and the second recessions comprise an upper part and a bottom part. The upper part has the shape of a truncated cone with an opening angle of 12° to 60°, in particular 12° to 36 °, and the ratio of the depth of the upper part to the diameter of the widest end of the upper part is at least 1.5 : 1.

The recessions can also be called structures of the structured surface, micro-cavity, microwell, conical recession and/or indentation.
The structured surface can be equipped as the device described in WO 2014/072432, which is hereby incorporated by reference. The main difference between the device disclosed in WO 2014/072432 and the present invention is that the present invention utilized rotation/centrifugation induced sedimentation for seeding and/or compacting the cells in the recessions, whereas in WO 2014/072432 unidirectional sedimentation by gravity is required.
Due to the shape of the structured surface "gravity" generated by rotation/centrifugation can be used effectively when distributing a cell suspension over the cell culture device leading to a fast and proper movement of the single cells into at least one of the first and second recession in a uniform or at least substantially uniform manner. In the context of the present invention, said distribution of the cells will also be referred to herein as loading one or more recessions with cells. A major benefit of the invention is that due to the sloped surface it can advantageously be avoided that cells sediment and remain on that surface, so that all or substantially all cells of the cell suspension are properly distributed into the desired recessions. Therefore, it is possible to generate and cultivate uniform or substantially uniform cell aggregates, namely 3D cell aggregates. This is of importance when it comes to different kinds of analysis or substance testing. Since the cultivation of very uneven 3D cell aggregates is a major problem in the state of the ad the device of the invention provides a simple but superior solution to this problem.
The sloped surface is in particular a surface which does not allow cells to sediment on. This is achieved by the shape of the surface. "Sloped" in terms of the invention in particular refers to curved, convex, declining or bent surfaces. In contrast, a "horizontal surface" describes a surface which is parallel to the wall, the bottom and/or the lid of the container, which is not desired in the invention.
The structured surface can be ideal not only for generating but also for cultivation cell aggregates. Devices described in the state of the art are either not deep enough or have an opening angle which is too wide for a robust 3D cultivation of cells. Therefore, it is for example not possible to change the cell culture medium without flushing cells out of the device or transferring them into another recession.
The opening angles can be 12° to 60°, in particular 12 to 36°, in particular 15 to 30°, in particular 20 to 25°. A large opening angle might be utilized as the acting (g-)force is generated by rotation of the container, as the cell aggregates are not likely to move out of the structured surface as long as rotational g-forces act on them. Furthermore, a wider opening angle might facilitate release and harvesting of cell aggregates from the device.

The ratio of the depth of the upper part to the diameter of the widest end of the upper part can be at least 1.5:1, in particular 2.5:1, in particular 2:1. It is surprising that a ratio of essentially 2:1 leads to a geometry which is superior to the known devices. It is also possible that the ratio is 4:1, 5:1 or at least 10:1. The skilled person is able to choose the best suited ratio for each approach.

The device allows for the production of a microstructured 3D cell aggregates and enabled for an improved cell analysis with proper dimensions and desired precision. Different further aspects of the geometry and shape of the recessions and surface can be added as described in this text. As will become clear from further exemplary embodiments, different aspects of the device can be combined with the feature that the surface between the two recessions causes a transport of cells by rotation/centrifugation induced sedimentation from surface into at least one of the first recession and the second recession. In other words, the surface between the first recession and the second recession is sloped and in particular does not have any "horizontal" parts or portions - meaning: is free from "horizontal" parts or portions, but in particular has only sloped or curved elements. In particular, the device of the present invention can be used with cells provided as a cell suspension.

Being free of any "horizontal" parts or portions in terms of the invention means in particular that the surface is shaped in a way that cells, in particular "sedimenting" cells in cell culture medium will not remain on that surface because of the sloped and/or curved shape.

Cells, which have been loaded into at least the first recession, are present in the bottom part of said recession in a manner, which forces the generation of 3D cell aggregates. Thus, the cells are present therein in a stacked and tight manner and separated from cells present e.g. in a second recession. This results in the formation of cell-cell contacts among the cells present in said recession and the formation of a three-dimensional cell aggregate in said recession, wherein only the outermost cells are directly exposed to the medium comprising inter alia nutrients. The structured surface can be made of a hydrogel substrate and is permeable e.g. for nutrients or other compounds comprised in the medium, there is, however, no limitation with respect to the medium and its ingredients.
The structured surface can be made of a hydrogel which is cell repellent. This improves the formation of uniform cell aggregates because all cells or nearly all cells reach the bottom part of one recession. Single cells do not attach to the sidewalls or the bottom of the recessions. In combination with the sloped surface this feature supports the uniformity of the generated 3D cell aggregates.

The part between two or more recessions can also be called partition wall. The partition wall can have a shape which facilitates a transport of cells by rotation/centrifugation induced sedimentation from a distal end of the partition wall into at least one of the first recession and the second recession. The distal end of the partition wall is also part of the sloped surface, in particular without any horizontal parts.

The size and the form of the 3D cell aggregates can be defined by the geometrical settings of the recession (such as e.g. the shape of the bottom part and the width of the recession). If a cell culture device comprises not only one but several recessions, which are identical in their geometrical settings, uniform 3D cell aggregates can be obtained in said recessions if such a device is loaded with a cell suspension.

The structured surface can provide for the advantage that the rotation/centrifugation induced sedimentation and/or accumulation and/or compacting of cells between the first and the second recession is avoided. Due to the sloped surface, in particular without any horizontal parts, cells get automatically transported into the desired direction. The surface can comprise at least one sloped sidewall. The sloped sidewall can be directed into one recession; therefore, the sloped sidewall is inwardly directed to one recession. The surface can be between at least two, in particular between four recessions, is shaped as a tapered tip. For example, this geometry can be provided for a cell culture device in which adjacent or neighboured surface parts between recessions are distanced from each other by, for example, 1 mm or 2 mm, or more than 2 mm, or below 1 mm. Also, other dimensions are possible. Furthermore, a width of the recession in the range of a single cell, which typically has a diameter in the range of between 0.008 mm and 0.02 mm, can be provided, as well as larger diameters such as e.g. 0.1 mm, or 0.2 mm or 0.3 mm or 0.5 mm.

The surface on the structured surface can comprise convex areas, which prevents the "sedimentation" of cells between two recessions.

Generally, the width of a recession depends on the type of cells to be analysed and the goal of the analysis: if cell small 3D cell aggregates, consisting of only a few cells, are to be analysed, the width of a recession should be rather small (this may apply for example to assays, wherein stem cells or progenitor cells are seeded in a clonal density). If larger 3D cell aggregates should be generated and/or analysed, a larger width should be applied (this may apply for example to assays, wherein chondrocytes are tested; these cells require to be present in large aggregates for proper differentiation and proper production of extracellular matrix). Further, the size of the cells to be analysed needs to be taken into account with this respect. Suitable widths range from container 10 micrometre to 1 mm. In this context width refers to the width of the bottom part of a recession.

The distance between the recessions can also depend on the type of cells to be used and/or analysed, but further on the size of the cell aggregates: the larger the 3D cell aggregates, the more supply with nutrients etc. comprised in the medium is needed. If the distance between the recessions is small, supply-problems may arise. Thus, in case of large 3D cell aggregates, it may be more appropriate to select a larger distance between the recessions (resulting in broader partition walls between the recessions). If slow-growing cells are used, supply might not be limiting; thus, small distances between the recessions may then be used.
Furthermore, the number of cells in the cell suspension used for loading the device can be considered: the higher the number of cells, the higher the consumption of nutrients etc. such that a larger width and distance may be more appropriate.
The following distances between the recessions and widths of a recession can be used: distances ranging from 0.25 mm (small cell aggregates, slow-growing cells) to 5 mm (larger cell aggregates, fast-growing cells), wherein the width of a recession may be in the range of 10 micrometre (very few cells in suspension) to 1 mm.
The following dimensions have been found as being suitable for most of the experimental approaches using standard cell lines: 1 mm or 2 mm distance between the recessions and 0.2 mm width per recession.

The structured surface can comprise at least one sloped sidewall. The term "slope" can be understood synonymously to steepness or gradient. This inwardly sloped sidewall can create the effect that cells are transported into at least one of the first recession and the second recession by the rotational/centrifugation force. A surface between two or more recessions can comprise adjacently positioned inwardly sloped sidewalls, such that a tip at the distal end of the surface is provided. The distance between two tips can be 1 millimetre (mm). However, also larger distances or also smaller distances are possible.

The truncated cone of the upper part of at least one recession can be steeper than the sloped sidewall. This feature additionally supports the flow of the cells into the bottom part of the recessions. The recessions can be conically shaped such that the cells, which are inserted in the respective recession, accumulate at the lowest end of the recession. Such a shape of the recession can be provided in the structured surface.

The conical shape of the recession can be provided such that the recession is tapered towards the base and thus towards the proximal end of the recession. Furthermore, the bottom of the first and/or the second recession can be flat, tapered or concave. In other words, the first and the second recession (and not only the upper part of the recessions) have a shape of a truncated cone. The truncated cone can have an opening angle of 25 degrees. Generally, the opening angle can be in a range of between 12 and 60 degrees, in particular 12-36 degrees. The sidewalls of a recession in the form of a truncated cone can have a defined slope. Generally, this slope can be steeper than the sloped sidewalls of the surface. The shape of a truncated cone is advantageous if a simple optical readout is desired in order to determine the volume of the individual 3D cell aggregates.
The conical shape of recession allows to cultivate cells that do not form any spheroids. For these cells, the recessions can be used as a measuring cup. Due to the defined shape, it is possible to analyse the volume of such 3D cell aggregates in an advantageously simple way. An opening angle between 12 and 60°, in particular 12°-36°, can be suitable for the use as a cell aggregate volumetry unit, similar to the use of a measuring cup.
The structured surface can be microstructured. The structured surface can be made of agarose, alginate or chemical modifications or mixture thereof. This can allow for tuning the mechanical properties and the permeability of the structured surface to a desired extent. Other biocompatible hydrogels might be used as well.
The structured surface can be free from polyacrylamide and may not formed from polyacrylamide, respectively, due to the toxic characteristics of acrylamide.

Furthermore, the structured surface might also be made from a microporous, cell-repellent material, in which the pores are much smaller than cells, so that cells will be supplied with nutrients in a similar way as by a hydrogel.

The sloped sidewall can have an angle which is selected from the group of angle ranges comprising 20 to 70 degrees, 30 to 60 degrees, 35 to 55 degrees, and 40 to 50 degrees. An angle of 40 degrees may be preferred. Moreover, said angle of the sloped side wall is defined relative to the wall of the container.

The structured surface can be removably positioned in the container. The removably positionable structured surface can be attached to the wall of the container. The structured surface can be designed as a cartridge that is mountable to the wall of the container. The structured surface might be attached to the container wall by adhesion and/or mechanical restrains. The structured surface night be clamped into the container to the wall.

The removably positionable structured surface can be attached to the bottom and/or the lid of the container. The structured surface can be designed as a cartridge that is mountable to the bottom and/or the lid of the container.
The structured surface can be an interchangeable insert. This enables the reuse of the container with an exchanged (fresh) structured surface. In a different embodiment of the invention the structured surface is fixed to the inner wall of the container.
The cartridge can comprise a structured surface. The cartridge can have at least two states. A mounted state which may also be called positioned state and/or attached state. A further state of the cartridge is a delivery state in which the cartridge might be delivered. The delivery state might be essentially different and/or similar to the mounted state.
The cartridge can comprise structures for receiving the cells. These structures are designed such that in the mounted state the structures correspond to the structures of the device as described above.

The device can comprise at least one of the following features:
- the removable structured surface is positioned at the inner surface of the container;
- the structured surface is positioned at the wall;
- the device may further comprise means for sterilisation and/or cleaning of the container.
The in particular the means for sterilisation and/or cleaning of the container might be a UV-lamp or rather a UV light sterilizer, in particular a UV-sterilization lamp, UV-germicidal lamp, UV-disinfection lamp and/or portable UV light sterilizer. This enables to sterilize the cavity of the container before or rather when exchanging the structured surface.

The cartridge for being mounted in a device as described above can be is mountable in the container. The cartridge can comprise the structured surface for receiving the cells.

The above described device can be used at least for generating, maintaining and/or harvesting of 3D cell aggregates. The 3D cell aggregates can be uniformly structured e.g. with respect to size and/or shape of the aggregates.
With WiDr cells, uniformity between single aggregates can 98% or more. Similar results were achieved with LNCaP, A-549, BT-474, HT-29, HuH7, MDA-MB 231 and MIA PaCa-2 cells. Inter-device variance can be between 1 - 5%.

The above described device can be used at least for the production of conditioned medium as described in above and below.

The above described device can also be used for production of extracellular vesicles (EVs). Medium can be circulated into a harvesting unit, in which EVs are separated via ultracentrifugation, filtration and/or other means for separation. This way, a continuous production unit for EVs can be created.

A further aspect of the invention is a method to generate 3D cell aggregates comprising the steps of:
- providing a device for seeding cells, in particular a device for seeding cells as described above;
- providing a cell suspension to be introduced in the container;
- loading the device with the cell suspension;
- rotating the container to exert a (g-)force on the/in direction of the structured surface and collecting cells in the structured surface;
- culturing the collected cells in the structured surface while further rotating the container.
This enables for the growth of 3D cell aggregates in the structures of the structured surface from the collected cells. The method may also comprise the step of letting 3D cell aggregates grow.
Due to the rotation of the container and the exertion of g-force in direction of the structured surface the unidirectional sedimentation by gravity can be replaced by the applied g-forces and the resulting compacting of the cells. The g-force being a unidirectional centrifugal g-force.

The method enables for a continuous supply of nutrients and other compounds to the cells.
In an embodiment of the invention a hydrogel is used in the structured surface.

The g-force applied to the cells while culturing can be essentially 1 g. This enables to grow 3D cell aggregates under conditions similar to standard gravitation, also called standard conditions in this context. It is also possible to apply different g-forces than 1 g while culturing depending on the requirements and the cells.
During seeding, meaning during collecting, of the cells different rotational speeds can be applied. After the seeding the rotational speed can be reduced to about 1 g.

The method can further comprise the step of maintaining 3D cell aggregates. The 3D cell aggregates can be obtained from the collected/seeded cells on/in the structured surface. The maintaining of the 3D cell aggregates can be performed similar as the culturing of the seeded cells. The g-force applied by the device can be approximately 1 g to carry out the maintenance or rather grooming, cultivation, or nursing of the 3D cell aggregates.

In other words: During seeding the rotational speed can be varied. After seeding the rotational seed can be adjusted to exert a force of 1 g onto the cells in the structures of the structured surface to allow for standard conditions (conditions similar to standard gravitation - 1 g) for the cells to grow. Accordingly, a variation of the g-forces might be advantageous while seeding the cells in the structures of the structured surface. Furthermore, for maintaining respectively culturing of 3D cell aggregates g-forces corresponding to required standard conditions (conditions similar to standard gravitation - 1 g) are exerted onto the cells.

As already described above the container can rotate while loading. This can further improve the homogeneity of the suspension while loading. The homogeneity of the cell suspension might be a pre-condition for obtaining a homogenous distribution of cells in the structured surface and/or homogenous/uniform 3D cell aggregates.

As described above the culture medium can be exchanged in the container. The exchange of culture medium can be performed at least in one of the following manners: in an automated way, a continuous way or as intermitted exchange.

The exchange of the medium can improve the nutrient supply of cells compared to static 3D cell cultures.

The device can be equipped to monitor the nutrient concentration of the culture medium. The device can comprise a sensor to monitor the nutrient level/concentration of the culture medium, in particular the concentration of glucose, amino acids in the culture medium, catabolites, or the pH-value.
In an embodiment the culture medium is exchanged once a predefined (low) nutrient concentration is reached or the monitored nutrient concentration falls below the predefined concentration. While exchanging the culture medium the nutrient concentration is further monitored. Fresh culture medium is added until a certain (further) predefined nutrient content/concentration is reached.

As described above g-forces of at least 0.1 g can be exerted in direction of the structured surface. The g-force exerted in direction of the structured surface can be at least one of at least 0.5, at least 1 g, at least 5 g, at least 10 g, and at least 300 g, at least 5000 g, depending on the requirement of the cells to be grown and the maintenance of the 3D cell aggregates.
A method for generating 3D cell aggregates in a serial reactor, wherein the provided device can be a first device. The method can comprise the steps of:
- providing a second device
- connecting the first device to the second device as a serial reactor.
The second device can be a device as described above. The second device can comprise a second container with a second wall, a second bottom and a second lid. The second container can comprise a second rotation axis, wherein the device is equipped to rotate the second container around the second rotation axis, by means for rotating the container about said rotation axis. The second container can comprise a structured surface, wherein the structured surface is equipped to receive cell. The structured surface can be arranged essentially parallel to the rotation axis.

The rotation can exert a (g-)force (meaning gravitational force) on the/in direction of the structured surface.

The rotation axis of the first device can equal to the second rotation axis of the second device. The serial reactor can be a serial reactor as described above.

The method can further comprise the step of loading the second device with a cell suspension. The second container may rotate while loading. The second device can be loaded simultaneously with the first device and/or separately after the first device and connected in series subsequently.
The cell suspension loaded in the first device and in the second device can be identical and/or different from each other.

The coupled/connected devices may comprise the same type and size of cell aggregates, but may also comprise different types of cells, so that the system represents a 'distance coculture'. Such a setup may be advantageous to produce conditioned cell culture medium containing factors from different organ origins. Furthermore, the factors of the first device may be beneficial to the aggregation of cells in the second device and therefore be an essential component to push the cell loaded in the device into aggregates.
The method can further comprise the step of rotating the serial reactor. The container of the first device and the second container can rotate at the same and/or a different speed. The speed of rotation (tangential speed) depends on the diameter of the container. Accordingly, the containers of different diameter might rotate at different speed to exert a similar g-force in direction of the structured surface. Furthermore, the rotation speed can be adjusted, depending on the loading of the device, e.g. the kind of cells or the number of cells loaded. As described above the container can rotate while loading.

The method can further comprise the step of harvesting the 3D cell aggregates. The harvesting can comprise at least one of the following steps:
- removing the 3D cell aggregates from the structured surface;
- collecting the 3D cell aggregates floating in the culture media.
The removal of the 3D cell aggregates from the structured surface can comprise at least one of the following steps:
- reduction and/or stopping the rotation of the container;
- swivelling or rather sluing of the container; and
- shaking or rather vibrating the container.
Due to this the 3D cell aggregates can escape and/or leave and/or exit the structures of the structured surface. The 3D cell aggregates in the culture medium can be extracted from the container together with the culture medium.

A method for producing conditioned medium produced by 3D cell aggregates can comprise the steps of:
- generating 3D cell aggregates as described above; and
- maintaining or rather culturing the 3D cell aggregates in the container; and
- collecting the culture medium surrounding the 3D cell aggregates form the container as conditioned medium.
The collection of the medium can be the removal and/or extraction of the culture medium from the container. The culture medium might be exchanged in the container to produce additional conditioned medium with the already generated 3D cell aggregates. The collection of the culture medium can be performed in a continuous and/or intermitted manner.
Conditioned medium is a spent media harvested from cultured cells. It comprises metabolites, growth factors, and extracellular matrix proteins secreted into the medium by the cultured 3D cell aggregates. Examples of each might include: metabolites such as glucose, amino acids, and nucleosides; growth factors such as interleukins, EGF (epidermal growth factor), and PDGF (platelet-derived growth factor); and matrix proteins such as collagen, fibronectin, and various proteoglycans. Most cell lines requiring conditioned medium are dependent on at least one major constituent found in the conditioned medium, as defined by American Type Culture Collection - ATCC (https://www.lgcstandards-atcc.org).

Further preferred embodiments are evident from the dependent patent claims. Features of the method claims may be combined with features of the device claims and vice versa.

The subject matter of the invention will be explained in more detail in the following text with reference to exemplary embodiments which are illustrated in the attached drawings, in which:
- Figure 1: shows a cross-section of a device for seeding cells;
- Figure 2: shows a longitudinal section of the device;
- Figure 3: shows a three-dimensional view of cross-section of the device;
- Figure 4: shows a cross section of the device immediately after being filled with cell suspension;
- Figure 5: shows a cross section of the device after compacting of the cells by rotation is complete;
- Figure 6: shows the process of 3D cell aggregate formation;
- Figure 7: shows the process of harvesting the 3D cell aggregates;
- Figure 8: shows a serial reactor comprising two connected devices;
- Figure 9: shows the distribution of cells in the structured surface;
- Figure 10: schematically shows the structured surface; and
- Figure 11: shows a longitudinal section of the container comprising an inner tubing.

The reference symbols used in the drawings, and their meanings, are listed in summary form in the list of reference symbols. In principle, identical parts are provided with the same reference symbols in the figures.

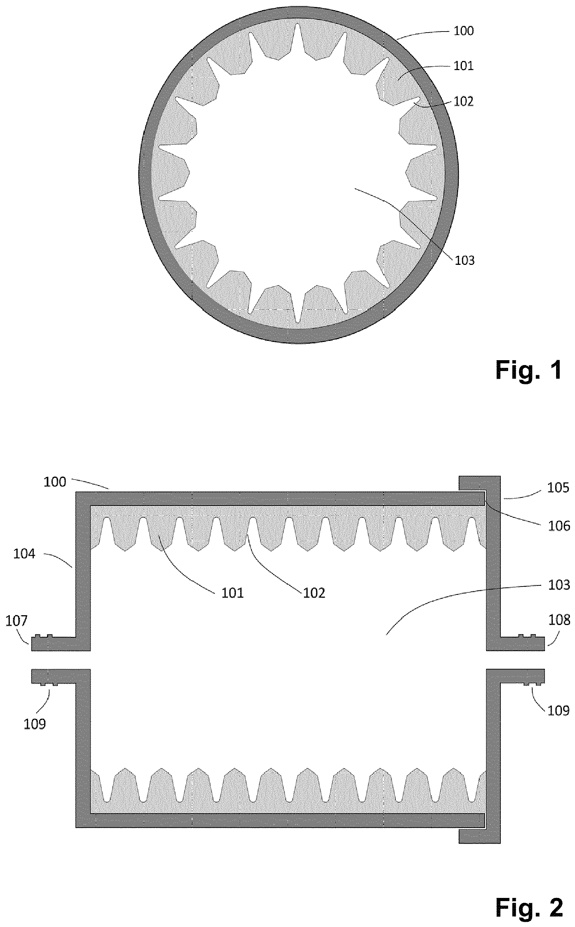

**Fig. 1** shows a cross-section of a device for seeding cells with a cylindrical container 10. The cylindrical container 10 comprises a wall 100, a bottom 104 and a lid 105. The wall 100 is containing a structured surface 101 with multiple conical recessions 102 or micro-wells. A cavity 103 is formed by the container 10 circumscribed by the wall, the bottom and the lid (see Figure 2).

**Fig. 2** shows a longitudinal section of a cylindrical device. It is designed as cylindrical tube. One side of the wall 100 is closed by a bottom 104. The bottom might be flat or structured. On the opposite side of the bottom, a lid 105 is serving to close the wall in a way that a junction 106 between wall 100 and lid 105 is air- and waterproof. This is either achieved in a permanent way, e.g. by welding or gluing the lid to the wall, or in a removable way e.g. by screwing or any other known way of connecting a lid to a cavity, as e.g. a bayonet lock or flange.
On one hand, the use of a removable lid may support multiple uses of the wall 100 and the lid. The structured surface attached to the wall 100 would have to be replaced in the multi-use operation. On the other hand, a permanent closure would imply a sterile single use device.
As shown in Fig. 1, the inner surface of the wall 100 is essentially entirely covered with a structured surface 101 with multiple conical recessions 102 or micro wells. The micro well can have any desired form required to maintain desired 3D cell aggregates. The cavity 103 is accessible via two ports (107 and 108). Each of the two ports carries a closing mechanism as e.g. thread or a valve, so that both sides of the device may be either connected with a tubing system for gas and liquid transfer or be closed with a plug, e.g a screw plug.

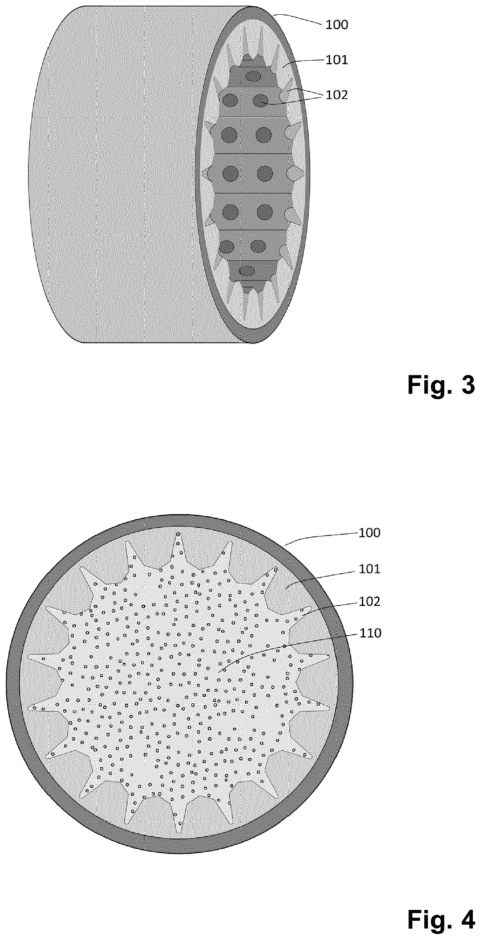

**Fig. 3** shows a three-dimensional view of cross-section. The entire inner surface of the wall 100 is covered with structured surface 101 comprising conical micro wells or recessions 102.

**Fig. 4** shows a cross section of the device immediately after being loaded with cell suspension 110. Rotation has not started yet and cells have not sedimented/compacted.

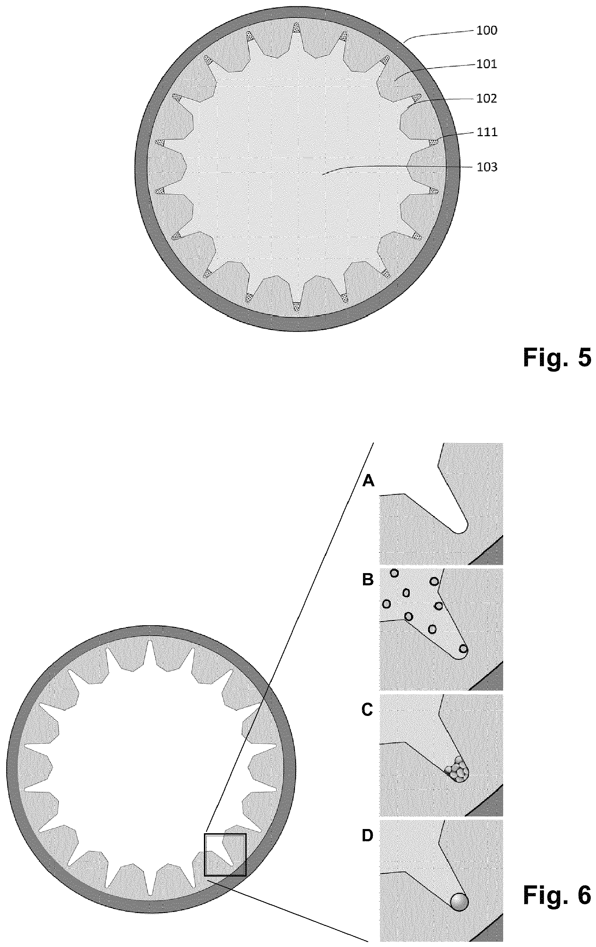

**Fig. 5** shows a cross section of the device after sedimentation/compacting by rotation is complete, all cells have formed aggregates 111 at the bottom of micro wells 102. The cavity 103 of device is now containing essentially cell-free culture medium.

**Fig. 6** shows the process of 3D cell aggregate formation in detail. The 3D cell aggregates can also be called spheroids. Insert A shows the device before filling with cell suspension. Insert B shows the device immediately after being loaded with cell suspension, as depicted in Fig. 4. Insert C shows that the cells have sedimented/compacted into micro-wells by rotation but not yet aggregated. Insert D shows that in each micro-well, cells have formed a 3D cell aggregates or spheroid.

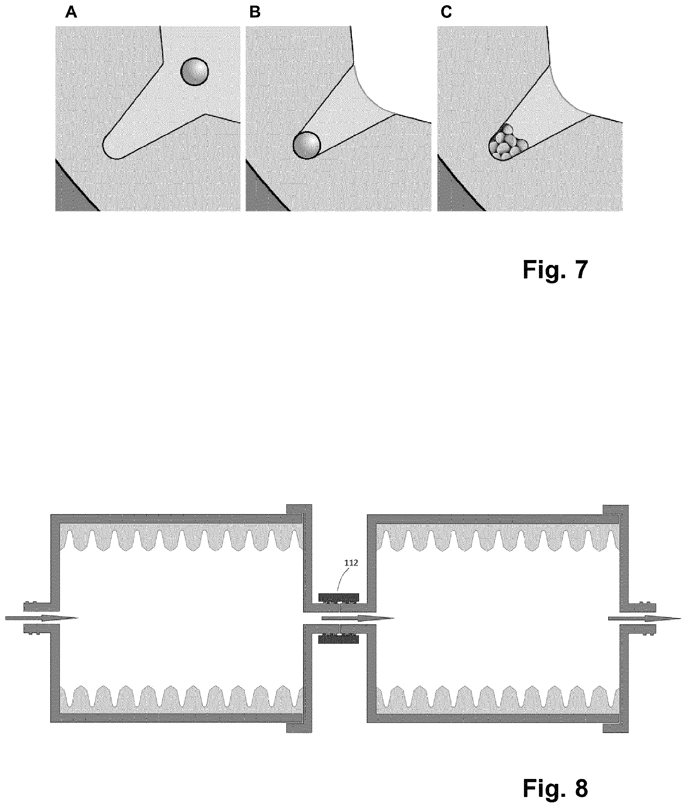

**Fig. 7** depicts the process of harvesting the 3D cell aggregates. A centrifugal force keeps cells in the micro-wells. When medium is removed while device is rotating, cells (C) and 3D cell aggregates/spheroids (B) remain trapped in the micro-wells due to surface tension of the air-liquid interface, even if rotation is stopped. For harvesting, 3D cell aggregates are flushed out of the micro-wells by shaking of device (A). For harvesting of the conditioned medium the 3D cell aggregates 111 remain in the microwells with and/or without rotating the container 10.

**Fig. 8** shows two devices coupled with an adaptor 112 to provide a serial reactor. The adaptor 112 enables the connection between the two devices. In the connected serial reactor culture medium can flow (arrow) from one device into another. It is also possible to couple several devices together. The shown devices are coupled in the lid-bottom manner. It might also be possible to connect the devices in a bottom-bottom or lid-lid manner. The type of coupling between the devices with the adaptor depends of the type of closing mechanism provided at the lid and/or bottom.

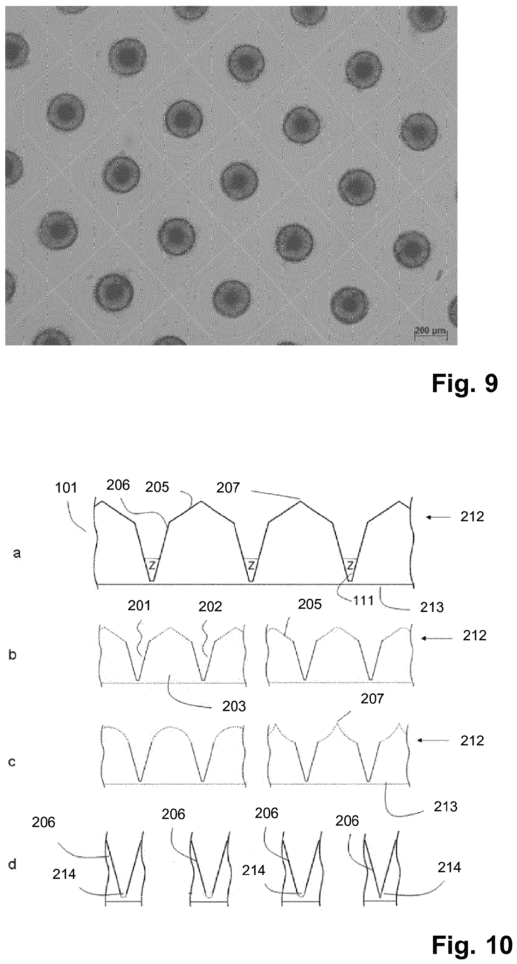

**Fig.9** shows the uniform distribution of cells in the structured surface 101. The 3D cell aggregates are uniformly structured with respect to size and shape. The cells are WiDr cells. 500 cells per microwell were seeded in the container. The 3D cell aggregates 111 were grown for 7 days in culture medium. The obtained 3D cell aggregates 111 are very evenly developed after seeding. The developed/grown 3D cell aggregates 111 reveal a homogenous and/or uniform distribution in terms of size and shape.

**Fig.10** schematically show the structured surface 101. Fig. 10 shows several structured surfaces 101 for generating and cultivating or cell aggregates 111, which comprises a first recession 201 for receiving cells and a second recession 202 for receiving cells. Furthermore, the structured surface 101 comprises a partition wall 203, which separates the first and the second recession and a bottom part 213. As can be gathered from Fig. 1 a - c, the "upper surface" 212 of the structured surface 101 has a shape, which facilitates a transport of cells from a distal end (" upper surface" 212) of the structured surface 101 into at least one of the first recession and the second recessions. Fig. 10 a and b show, that the surface can be shaped like a sloped sidewall 205 which causes transport effect. These sidewalls can end in a tip 207. But other shapes are also possible as e.g. a convex shape or a curved shape. Consequently, the structured surface 101 of Fig. 10 facilitate a high throughput uniform cell distribution within the structured surface 101 and advantageously avoid sedimentation of cells at undesired locations, e.g. the upper surface or recession sidewall. As can be gathered from Fig. 10, the structured surface 101 does not comprise any "horizontal" section or any "horizontal" portion, where the cells could disadvantageously sediment. Fig. 10 d shows different possibilities for the bottom part 214 of the recessions. The upper part 206 is preferably always shaped as a truncated cone. The bottom part can have various geometries. E.g. the bottom part can just be flat like in Fig. 10 a - c. In this case the bottom part is the apical end of the upper part.
Furthermore, the partition wall 203 provides for a section 206 between the distal end and the base section 104. Section 206 comprises a second slope (truncated cone) and it can be seen that the second slope is steeper than the slope of sidewall 205 of the "upper surface" portion 112. Therefore, the cell transport to the bottom of the structured surface is provided. The structured surface 101 of Fig. 10 a comprises a partition wall with a tapered surface ending in a tip 207 at the distal end of the partition wall 203. Furthermore, the recession can comprise a tapered bottom part as shown in Fig 1 d (3. and 4. figure from the left). Providing such geometry of the device on the given scale of structured surface 101 facilitates and improves the formation of 3D cell aggregates 111, their cultivation as well as their analysis.
As can e.g. be gathered from Figs. 1, a raster of conical recessions microwells with respective chamfers at the opening, i.e. distal end, of the recessions, and a tapered bottom is presented. The depth of the conical recessions allows for an uncomplicated change of the cell culture medium, the transfer into another culture dish, and the staining of 3D cell aggregates 111 without flushing the aggregates out of the structured surface 101.

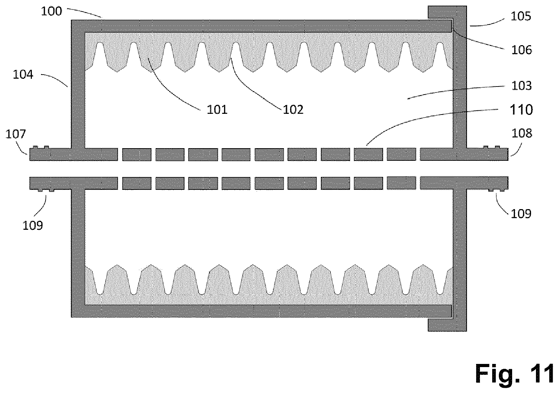

**Fig.11** shows a longitudinal section of the container 10 comprising an inner tubing 110. The inner tubing reaches though the container 10 from the bottom 104 to the lid 105. Cells can be homogenously emitted from inner tubing 110 towards the structured surface 101.
The inner tubing 110 is positioned inside the container 10. The inner tubing 110 is surrounded by the wall, the bottom and the lid. In other wards: The inner tubing 110 is an additional cavity inside the container 10.
The inner tubing 110 essentially extends from the bottom 104 to the lid 105. The inner tubing 110 can be punctured and/or perforated. The cells can be injected inside the container 10 via the inner tubing 110.
The inner tubing 110 can rotate together with and/or in a different speed and/or in a different direction than the container 10. In another embodiment of the invention the inner tubing 110 is stationary and does not rotate.

While the invention has been described in present preferred embodiments of the invention, it is distinctly understood that the invention is not limited thereto, but may be otherwise variously embodied and practised within the scope of the claims.

## Claims

1. Device for seeding cells, wherein the device comprises
a container (10) with a wall (100), a bottom (104) and a lid (105);
wherein the container comprises a rotation axis;
wherein the device is equipped to rotate the container (10) around the rotation axis (120), by means for rotating the container about said rotation axis;
**characterized in that**
the container comprises a structured surface (101),
wherein the structured surface is equipped to receive cells, and
wherein a rotation around the rotation axis (120) exerts a (g-)force in direction of the structured surface (101).

2. Device according to claim 1, wherein the structured surface (101) comprises a hydrogel and/or wherein the structured surface (101) comprises micro wells for receiving cells.

3. Device according to one of claims 1-2, wherein the device is equipped to promote the growth of 3D cell aggregates (111), in particular the device is equipped such that the structured surface (101) covers essentially the whole wall (100) of the container (10) and/or such that at least the portion of the wall (100) not covered by the structured surface (101) is coated with a cell repellent.

4. Device according to one of claims 1-3, wherein the structured surface (101) is arranged essentially parallel to the rotation axis (120).

5. Device according to one of claims 1-4, wherein the container (10) comprises an inner tubing (110), wherein cells are homogenously emitted from the inner tubing towards the structured surface (101).

6. Device according to one of claims 1-5, wherein container (10) is symmetrical with respect to the rotation axis (120),
in particular wherein the container (10) comprises at least one of the following features:
- the wall (100) of the container (10) is curved;
- the structured surface (101) is curved;
- the container (10) is cylindrical, conical and/or can have another regular or irregular shape;
- the device comprises a closing mechanism (109) to removable connect the bottom (104) and/or the lid (105) to the wall (100).

7. Serial reactor, wherein the serial reactor comprises a first device according to one of claims 1-6, a second device according to one of claims 1-6 and an adaptor (112); wherein the adaptor (112) is equipped to connect a first device to a second device.

8. Device according to one of claims 1-7, wherein the structured surface (101) comprises
a surface, at least a first recession and a second recession for receiving cells for generating and cultivating cell aggregates,
wherein the first and the second recessions are spatially separated, and wherein the surface between the first and the second recession is a sloped surface and
wherein the first and the second recessions comprise an upper part and a bottom part and
wherein the upper part has the shape of an truncated cone with an opening angle of 12° to 60°, in particular 12° to 36°, and the ratio of the depth of the upper part to the diameter of the widest end of the upper part is at least 1.5 : 1.

9. Device according to one of claims 1-8, wherein the structured surface (101) is removably positioned in the container (10),
wherein the structured surface (101) is designed as a cartridge that is mountable to the wall (100) of the container;
in particular wherein the device comprises at least one of the following features:
- the removable structured surface (101) is positioned at the inner surface of the container (10);
- the structured surface (101) is positioned at the wall (100);
- the device further comprises means for sterilisation of the container (10).

10. Cartridge for being mounted in a device according to one of the claims 1-9, wherein the cartridge is mountable in the container (10), and wherein the cartridge comprises the structured surface (101) for receiving the cells.

11. Use of the device according to one of claims 1 - 10, for generating, maintaining and/or harvesting of 3D cell aggregates (111) and/or for the production of conditioned medium.

12. Method to generate 3D cell aggregates (111) comprising the steps of:
- providing a device for seeding cells,
comprising a container (10) with a wall (100), a bottom (104) and a lid (105); wherein the container comprises a rotation axis
wherein the device is equipped to rotate the container around the rotation axis (120), by means for rotating the container about said rotation axis;
the container comprises a structured surface (101),
wherein the structured surface is equipped to receive cell, and
wherein the rotation exerts a (g-)force on the/in direction of the structured surface (101);
- providing a cell suspension to be introduced in the container (10);
- loading the device with the cell suspension;
- rotating the container (10) to exert a (g-)force on the/in direction of the structured surface (101) and collecting cells in the structured surface (101);
- culturing the collected cells in the structured surface (101) while further rotating the container (10);
in particular wherein the device is a device according to any of the claims 1-9.

13. Method according to claim 12, wherein 2D cell aggregate formation is promoted on an inner wall of the container 10 and the structured surface 101 is arranged on top of the 2D cell aggregates.

14. Method according to any one of claims 12-13, wherein the provided device is a first device, the method comprises the steps of:
- providing a second device, comprising a second container (10) with a second wall (100), a second bottom (104) and a second lid (105);
wherein the second container comprises a second rotation axis wherein the second device is equipped to rotate the second container around the second rotation axis (120), by means for rotating the container about said rotation axis;
the second container comprises a structured surface (101),
wherein the structured surface is equipped to receive cell, and wherein the structured surface (101) is arranged essentially parallel to the second rotation axis (120);
wherein the rotation exerts a (g-)force on the/in direction of the structured surface (101);
in particular wherein the second device is a device according to one of claims 1- 9,
- connecting the first device to the second device as a serial reactor;
- loading the second device with a cell suspension,
wherein the cell suspension loaded in the first device and in the second device is identical and/or different from each other;
- rotating the serial reactor, in particular wherein the container of the first device and the second container (10) rotate at the same and/or different speed.

15. Method for producing conditioned medium produced by 3D cell aggregates (111) comprising the steps of:
- generating 3D cell aggregates (111) according to one of the claims 12-14; and
- maintaining the 3D cell aggregates (111) in the container (10); and
- collecting the culture medium surrounding the 3D cell aggregates (111) form the container (10) as conditioned medium.
